# EUROPEAN PATENT SPECIFICATION

(11) **EP 3 480 310 B1**
(45) Date of publication and mention of the grant of the patent: **26.11.2025**
(21) Application number: 17819255.5
(22) Date of filing: 28.06.2017
(51) Int. Cl.: C12N 15/11, C12Q 1/68, C12Q 1/6811

(54) **PROBE FOR NUCLEIC ACID ENRICHMENT AND CAPTURE, AND DESIGN METHOD THEREOF**
SONDE ZUR NUKLEINSÄUREANREICHERUNG UND -ERFASSUNG UND ENTWURFSVERFAHREN DAFÜR
SONDE POUR L'ENRICHISSEMENT ET LA CAPTURE D'ACIDES NUCLÉIQUES, ET SON PROCÉDÉ DE CONCEPTION

(30) Priority: 30.06.2016 CN 201610504501
(43) Date of publication of application: 08.05.2019
(73) Proprietor: Amoy Diagnostics Co., Ltd, Xiamen, Fujian 361027 (CN)
(72) Inventor: SHI, Weijie, Xiamen Fujian 361027 (CN); LIN, Qinghua, Xiamen Fujian 361027 (CN); JI, Binfeng, Xiamen Fujian 361027 (CN); TANG, Zhenghua, Xiamen Fujian 361027 (CN); LI, Xuchao, Xiamen Fujian 361027 (CN); RUAN, Li, Xiamen Fujian 361027 (CN)
(74) Representative: Hoppe, Georg Johannes
(86) International application number: PCT/CN2017/090446
(87) International publication number: WO 2018/001258

(56) References cited:
- WO-A2-02/097390
- WO-A2-2004/104172
- WO-A2-2004/104172
- WO-A2-2005/045060
- WO-A2-2008/051604
- CN-A- 106 086 013
- CN-B- 1 882 703
- US-A- 6 083 699
- US-A1- 2004 229 269
- US-A1- 2006 172 325

## Description

### Technical Field

The invention relates to the field of gene sequencing, in particular to a kind of probes for nucleic acid enrichment and capture and a design method thereof.

### Background Technique

The sequences of the human genome are too large, so it is necessary to enrich the target gene in order to study the base mutation at a specific location in the target gene.PCR enrichment and hybridization enrichment are the most common enrichment methods.PCR enrichment mainly includes multiplex PCR and digital PCR.PCR has some limitations such as amplification bias, interaction of primers in multiplex amplification, and inability to detect unknown sequences (such as gene rearrangement). Hybridization enrichment mainly includes chip hybridization and solution hybridization. Hybridization is not suitable for small area enrichment, and requires more samples. Chip hybridization is an early technology that promotes hybridization reaction by increasing sample size,solution hybridization promotes the hybridization reaction by increasing the amount of the probes, reducing the amount of sample.By hybridization, specific probes were designed for target genes, and the target genes were captured by hybridization between probes and genomes.After enrichment, the mutation of the target genes can be analyzed by high throughput sequencing.

The main companies for capturing probes design and synthesis are Roche Sequencing, Inc., Agilent Technologies, Inc.,and IDT (Integrated DNA Technologies, Inc).The probes design is designed for single strands in the target area and is designed to be of different lengths (29-39 bp as described in patent US 7636637).Reference > 60 bp, https://lifescience.roche.com/wcsstore/RASCatalogAssetStore/Articles/BIOCHEMICA_3 _09_p13-14.pdf;>100 bp, as described in Agilent patent US20110184161;120 bp probes with different overlaps (e.g. 1x, 1.5x, 2x or 4x tiling) described in the literature PMID 19182786, PMID 19835619. The one-direction design of the probes makes it impossible to capture complementary strand nucleic acid libraries during the capture process, resulting in loss of capture copy number. In addition, the probes were designed according to the normal genome sequences, and the normal genome sequences rather than the mutant sequences are preferentially captured, resulting in the deviation of mutation frequency in the sequencing results.

A previous patent document of the prior art to be mentioned herein is US 2004/229269 (HASHMI, G. et al.), also seeking to detect known genetic mutations. Like in the underlying invention, this prior art provides sense strand probes and antisense strand probes and seeks to minimize interaction between both. This is achieved by distributing sense probes and complementary amplicon antisense strands into more than one different containers so as to perform separate hybridization reactions in different containers, said number of containers being as small as possible.

### Summary of the Invention

The object of the present invention is to provide a kind of probes for nucleic acid enrichment and capture, which can improve the capture specificity and increase the number of original copies of the nucleic acid captured from the sample.

To achieve the above objects, the present invention provides a kind of probes for nucleic acid enrichment and capture. The probes are two-direction probes, including sense strand probes and antisense strand probes. There is no overlap in sense strand probes or in antisense probes, the length of the probes is 30-89 bases. The 3'or 5' of the probes have a biotin label that can bind the avidin on magnetic beads.

Further, the sequences design method of the two-direction probes is as follows:
(1) the following length of reference sequences are selected according to the gene mutation point in the corresponding position of the reference genome:
   if they are point mutations, insertion mutations or deletion mutations on exons , the exon length should be selected;
   if they are the fusion mutatons the length of introns and exons at the fusion point should be selected;
(2) the reference sequences, namely the sense strand template, are taken to design equal length of sense strand probes without overlapping ;
(3) the reverse complementary sequences of the reference sequences, namely the antisense strand template , are taken to design equal length of antisense strand probes without overlapping ;the antisense strand probes without overlapping have the same length as the sense strand probes without overlapping, and are dislocated with the sense strand probes without overlapping :The length of the antisense strand probes without overlapping is n,if n is odd, the most suitable dislocation is (n+1) /2;if n is even, the most suitable dislocation is n/2;
(4) through software analysis, the probes containing repeat sequences are eliminated;the software analysis includes a probes sequences analysis and a target sequences analysis, the software analysis can adopt repeat sequences recognition software;
(5) according to the high-throughput sequencing results of DNA samples captured by probes, the captured non-specific fragments are compared with all probes, and the probes with matching results are eliminated;
(6) the capture probes which are ultimately applicable to the reference sequences are obtained.

Furthermore, in order to capture both wild type and mutant type sequences, specific probes are added, the sequences of the specific probes are as follows:mutation site upstream matching sequences, base free spacer groups and mutation site downstream matching sequences; the length of the specific probes sequences is n, and if n is odd, the length of the upstream matching sequences or the downstream matching sequences is (n+1)/2;if n is even, the length of upstream matching sequences or downstream matching sequences is n/2.

Further, for the mutation point of the mutation type sequences, the mutation bases at the mutation point are replaced by base free groups in the design of the specific probes.

In order to better capture the double-stranded genome target sequences, two-direction probes (designed sense strand probes and antisense strand probes, respectively) are designed based on the genome target sequences required for capture.The sense strand probes are probes without overlapping design, and the antisense strand probes are also probes without overlapping design. In order to reduce the complementary pairing between the sense strand and the antisense strand probes, the sense strand and the corresponding antisense strand probes are designed to be dislocated, that is, each sense strand and the corresponding antisense strand probes have a complementary part of half length of probes, which not only ensures the capture of the template double strand, but also minimizes the interaction between the sense strand probes and the antisense strand probes.

The length of the probes are 30-89 bases . The 3'or 5' of the probes have a biotin label that can bind the avidin on magnetic beads in order to capture target sequences in subsequent magnetic environments.

At the same time, in order to capture both wild type and mutant type sequences at the same time, special probes are added on the basis of the two-direction probes. The mutant sites in the specific probes are replaced by base free spacer groups (dSpacer, Spacer 9 or Spacer 18, etc.), and the mutant type and wild type share same flanking sequences which are on both sides of the base free spacer modified groups.

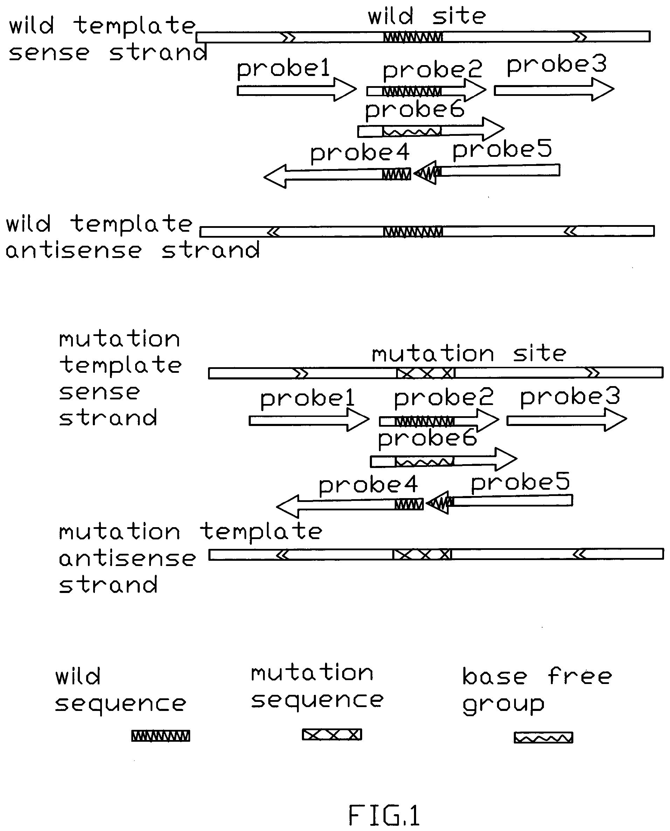

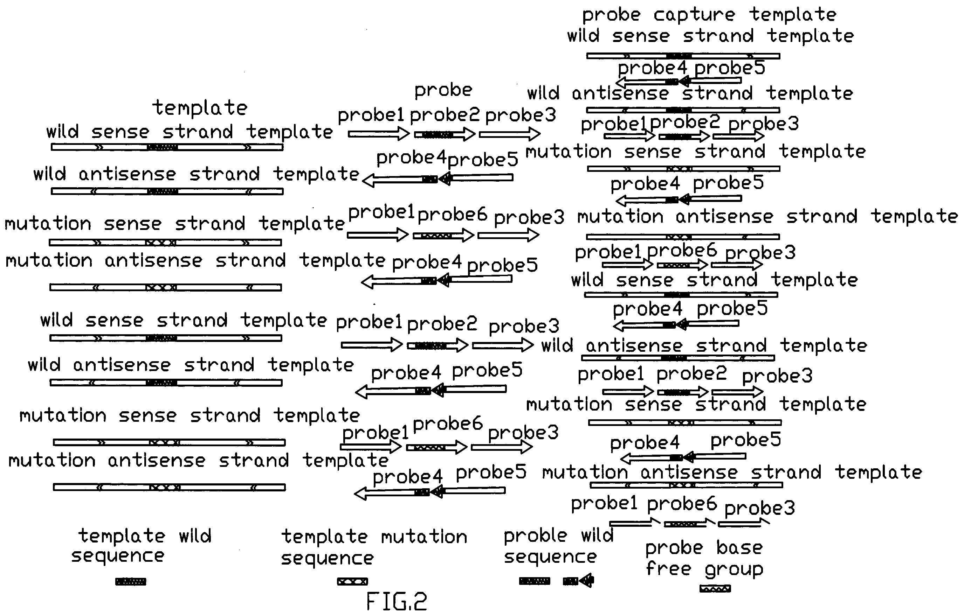

The design principle of the probes is shown in Fig. 1. According to the corresponding sites of gene mutation points in the human reference genome (e.g. Homo sapiens, Release 19 (GRCh37.p13)), the corresponding length reference sequences are selected for probes design. According to the complementary double strands of the genome, the probes without overlapping in the sense strand and the probes without overlapping in the antisense strand are designed respectively. Each sense strand probes are complementary to the antisense strand probes at the corresponding position with half the length of the probes, and each antisense strand probes are complementary to the sense strand probes at the corresponding position with half the length of the probes;and according to the desired detected genome mutations, we design bias free probes in the corresponding location. The hybridization capture of the designed probes with the sample DNA is shown in Fig. 2A,Fig. 2B,Fig. 2C.

The probes design steps:
1. According to the corresponding position of the gene mutation point in the human reference genome (such as Homo sapiens, Release 19 (GRCh37.p13)), the length of the corresponding length reference sequences are selected, if they are the point mutation, insertion mutation or deletion mutation in the exon, the length of the exon is selected;if they are the fusion mutation, the length of introns and exons at the fusion point should be selected.If the T790M point mutation of EGFR gene is observed, the exon 20 of EGFR gene is selected; if EGFR gene E746_A750del deletion mutation is observed, the exon 19 of EGFR gene is selected ;if the fusion occurs in the intron 19 of ALK gene, the exon 19, the intron 19 and the exon 20 of ALK gene are selected.
2, The reference sequences (sense strand template) are taken to design the same length of the sense strand probes without overlapping, using software (such as Array Designer) or manually.
3. The reverse complementary sequences (antisense strand template) of the reference sequences, are taken to design the same length of the antisense strand probes without overlapping.The antisense strand probes have the same length as the sense strand probes, and are dislocated with the sense strand probes. The probes design is by software (such as Array Designer) or manually. If the probe's length is n, the possibility of dislocation is 1~ (n-1). If n is odd, the most suitable dislocation is (n+1) /2;if n is even, the most suitable dislocation is n/2.This dislocation will minimize the interaction between the sense strand probes without overlapping and the antisense strand probes without overlapping.
4. Through software analysis or (and) test results, the probes containing repeat sequences are eliminated.The software analysis includes probes sequences analysis and target sequences analysis, the software analysis can adopt a repeat sequences recognition software , such as online software RepeatMasker, local software Blast and BWA. Test result, according to the high throughput sequencing results of DNA samples captured by probes, the captured non-specific fragments are compared with all probes(such as the local software), and the probes with matching results are eliminated;
5. The capture probes that are ultimately applicable to reference sequences are determined.

The principle of using the probes to hybridize and capture sample DNA is shown in Fig. 2A,Fig. 2B,Fig. 2C.Wild type sense strand template, wild type antisense strand template, mutant type sense strand template and mutant type antisense strand template are found in the DNA samples.The probes contain wild type sense strand probes, wild type antisense strand probes, unbiased sense strand probes and unbiased antisense strand probes. Under certain conditions, wild type antisense strand probes can hybridize and capture wild type sense strand templates, wild type sense strand probes can hybridize and capture wild type antisense strand templates, wild type antisense strand probes and unbiased antisense strand probes can hybridize and capture mutant sense strand templates, wild type sense strand probes and unbiased sense strand probes can hybridize and capture mutant antisense strand type template.

The two-direction probes of the invention can improve the capture specificity (i.e., reduce the capture of genome DNA at non-target locations), and increase the number of original copies of the nucleic acid captured from the sample.The two-direction probes and the unbiased probes containing the base free spacer groups of the invention can also significantly improve the capture of the sample mutant type DNA.

### Brief Description of the Drawings

Fig. 1 is a schematic diagram of the design principle of the probes of the invention.
Fig. 2 is a schematic diagram of the principle of hybridizing and capturing sample DNA using the probes of the invention.

### Detailed Description

Embodiments of the present invention are described in detail below, examples of which are illustrated in the drawings in which identical or similar labels from start to end denote identical or similar elements or elements having the same or similar functions.Embodiments described below with reference to the accompanying drawings are illustrative and intended to be used to interpret the present invention, not to be construed as limitations to it.If the specific technology or condition is not specified in embodiment, it shall be carried out in accordance with the technology or condition described in the literature in the art or in accordance with the product specification.All the reagents or instruments that are not indicated by the manufacturer are regular products that can be purchased through the market.

### Embodiment 1 Effect of the two-direction probes on the on Target rate of different GC content fragments

6 genome segments (Homo sapiens, Release 19 (GRCh37.p13) chr2:29446476-chr2:29446775;chr2:29447226-chr2:29447525 ; chr2:29448065-chr2:29448364 ; chr6:117642641-chr6:117642940 ; chr6:117645323-chr6:117645622 ; chr6:117641038-chr6:117641337) are taken to design the probes: one-direction probes 3 times overlapping coverage, or two-direction probes misalignment coverage;the probes length is 59bp, and the probes's 3 'end is Biotin modified.There are many specific probes sequences, Homo sapiens, Release 19 (GRCh37.p13) chr2:29446476-chr2:29446775 segment is taken as an example, five one-direction probes are listed as SEQ ID NO:1-5;five two-direction probes are listed as SEQ ID NO:6-10. 30 ng Leukocyte DNA is taken to build the library with KAPA kit, using hybridization reagents, a hybridization capture of 2h and 24h is performed, using Roche's SeqCap EZ Hybridization and Wash Kit according to the kit instructions.The results of sequencing are shown in Table 1.

**Table 1 Influence of the two-direction probes on the on Target rate of different GC content fragments**

| probe s cover age | Hybr idiza tion time | yield | Sequencin g data volume | onTarge t | Coverag e | Uniformit y | Depth | UID |
|---|---|---|---|---|---|---|---|---|
| one-di rectio n | 24h | 990 ng | 146.96Mb p | 5.36% | 100.00% | 100.00% | 3378. 06 | 2103. 65 |
| two-di rectio n | 24h | 1065 ng | 156.92Mb p | 9.45% | 100.00% | 99.11% | 6549. 81 | 2854. 83 |
| one-di rectio n | 2h | 1050 ng | 157.91 Mb p | 4.61% | 100.00% | 100.00% | 3140. 51 | 1902. 92 |
| two-di rectio n | 2h | 1140 ng | 162.28Mb p | 5.57% | 100.00% | 99.83% | 4023. 36 | 2088. 54 |

Referring to Table 1, the yield of the two-direction probes dislocation coverage (amplified library yield after capture) is significantly higher than that of one-direction overlap coverage, and the onTarget rate of hybridization capture is also significantly increased, and the effect of 24-hour hybridization group is more obvious than that of 2-hour hybridization group.Coverage is consistent in each group.The Uniformity of the two-direction probes is low.Because of the limited amount of sequencing data, the UID of the two-direction probes dislocation coverage increases with the increase of onTarget rate. Uniformity value, the two-direction probes is lower than the one-direction overlapping probes, mainly because the different sequences of each probes determines the deviation of the capture efficiency, so increasing the frequency of the probes coverage, can effectively reduce capture ability differences of the probes at different positions of the base , so that uniformity is better. one-direction design of the probes can not capture double-stranded nucleic acid libraries, resulting in loss of capture copy number, while the two-direction probes fills the corresponding loss, so that Depth and UID are greatly improved.Sequencing data is sometimes higher than a single item, sometimes lower than a single item, because while high throughput sequencing, sample quality is converted according to the expected amount of data, multiple samples are mixed and sequenced, so there may be deviations in the process of sampling and mixing, it can not guarantee absolute accuracy, in addition, when the sample is on the sequencing instrument it need PCR amplification process , it can not ensure that the amplification efficiency of each sample is exactly the same .

### Embodiment 2 Effects of the two-direction probes on the on Target rate of different chromosomes

5 genome segments Homo sapiens, Release 19 (GRCh37.p13) chr2:29446201-29448364, chr7:55241635-55241748, chr7:55242396-55242539, chr7:55249029-55249107, chr7:55259462-55259576 are taken to design the probes as follows; one-direction probes no overlap coverage and two-direction probes dislocation coverage.The probes length is 35-80bp and the probes Tm is 72-78 °C, and the probes 3 'end is Biotin modified.There are many specific probes. Homo sapiens, Release 19 (GRCh37.p13) chr2:29446201-29448364 is taken as an example, five one-direction probes are listed as SEQ ID NO:11-15. 5 two-direction probes are listed as SEQ ID NO:16-20, are showed in Table 2. 30 ng Leukocyte DNA is taken to build the library KAPA kit, using hybridization reagents, a hybridization capture of 2h is performed, using Roche's SeqCap EZ Hybridization and Wash Kit according to the kit instructions.The results of sequencing are shown in Table 2.

**Table 2 Influence of the two-direction probes on the on Target rate of different chromosomes**

| probes coverage | Hybri dizati on time | yiel d | Sequenc ing data volume | onTar get | Coverag e | Uniform ity | Depth | UID |
|---|---|---|---|---|---|---|---|---|
| one-direc tion | 24h | 798 ng | 167.21M | 3.95 % | 100.00% | 100.00 % | 2151.7 5 | 1272. 26 |
| two-direct ion | 24h | 896 ng | 156.20M | 10.95 % | 100.00% | 100.00 % | 5381.3 4 | 2690. 09 |

Refer to Table 2, the yield of two-direction probes dislocation coverage (amplified library yield after capture) is significantly higher than that of one-direction no overlap coverage, and the onTarget rate of hybridization capture is also significantly increased; Coverage and Uniformity are similar in all groups. Because of the limited amount of sequencing data, the UID of two-direction probes dislocation coverage increases with the increase of onTarget rate.

### Embodiment 3 Influence of the two-direction probes on UID

Several genome segments are selected for probes design, including the exon 31, the intron 31 and the exon 32 of ROS1 gene and the exon 19 of EGFR gene, the probes design is performed as follows : one-direction probes 3 times overlapping coverage, two-direction probes dislocation coverage, the probes length is 59 bp, the probes 3'end is modified by Biotin. The probes containing repeat sequences is eliminated by software analysis and experimental results. The specific probes sequences are more, taking the exon 19 of the EGFR gene as an example, Homo sapiens, Release 19 (GRCh37.p13) chr7:55242415-55242513, five one-direction probes, SEQ ID NO:21-25, which are ultimately applicable to the reference sequences, are listed. Four two-direction probes, SEQ ID NO:26-29, which are ultimately applicable to the reference sequences, are listed. Taking the exon 31, the intron 31 and the exon 32 of ROS1 gene as examples, Homo sapiens, Release 19 (GRCh37.p13) chr6:117650492-117658503. Five one-direction probes, SEQ ID NO:30-34, which are ultimately suitable for reference sequences, are listed. Five two-direction probes, SEQ ID NO:35-39, which are ultimately suitable for reference sequences, are listed. Five two-direction probes containing repeat sequences, SEQ ID NO:40-44, which are eliminated by software, are listed. Five two-direction probes containing repeat sequences, SEQ ID NO:45-49, which were eliminated from the test results, are listed , which are showed in the attached table. At the same time, an unbiased probes containing a base free spacer groups is designed for the mutation type NM_005228:exon 19:c.2235_2249del:p.745_750del of the EGFR with the exon 19 deleted to increase the capture of the mutant template.The unbiased probes is replaced by the base free spacer groups Spacer 18, the sequences of which is SEQ ID NO: 50-Spacer 18-SEQ ID NO: 51-3'-Biotin.
wherein, TGAGAAAGTTAAAATTCCCGTCGCTATCAA is SEQ ID NO:50;
CGAAAGCCAACAAGGAAATCCTCGATGTGA is SEQ ID NO:51.

Specifically is:
TGAGAAAGTTAAAATTCCCGTCGCTATCAA-Spacer18-CGAAAGCCAACAAG GAAATCCTCGATGTGA-3'-Biotin. 30 ng human Leukocyte DNA or Plasma DNA is taken to build the library with KAPA kit , using hybridization reagents, a double-hybridization capture of 24h + 24h is performed, using Roche's SeqCap EZ Hybridization and Wash Kit, according to the kit instructions. The results of sequencing are shown in Table 3.

**Table 3 Influence of the two-direction probes on UID**

| probes coverag e | Sampl e type | yield | Sequencin g data volume | onTarg et | Covera ge | Unifor mity | Depth | UID |
|---|---|---|---|---|---|---|---|---|
| one-dire ction | Leuco cyte DNA | 608 | 211.21M | 91.37 % | 100.00 % | 100.00 % | 2483.87 | 427 4.50 |
| two-dire | Leuco | 1022 | 212.95M | 93.31 | 100.00 | 100.00 | 2582.17 | 456 |
| ction | cyte DNA | | | % | % | % | | 4.90 |
| one-dire ction | Plasm a DNA | 630 | 233.69M | 92.14 % | 100.00 % | 100.00 % | 418.84 | 1140 .32 |
| two-dire ction | Plasm a DNA | 865 | 245.13M | 94.50 % | 100.00 % | 100.00 % | 481.23 | 128 7.16 |

Referring to Table 3, the yield of the two-direction probes dislocation coverage (the yield of the library amplified after capture) is significantly higher than that of the one-direction probes groups, and the onTarget rate of hybridization capture was slightly increased. Coverage and Uniformity are similar in all groups.The UID of leukocyte DNA and plasma DNA increase by 6.8% and 12.9% respectively, when the two-direction probes dislocation coverage group compares with the one-direction probes coverage group under the condition that the onTarget and the data amount are similar.The Depth of the two-direction probes is higher than that of the one-direction probes.It is concluded that the two-direction probes have better hybridization capture ability than the one-direction probes with 1, 2 or 3 times coverage.

The two-direction probes increase the coverage frequency of the probes to the target base, so a comparison is introduced to show that the two-direction probes are superior to the one-direction probes in design.

### Embodiment 4 Effects of the two-direction probes on mutation detection

Several genome segments were selected for probes design, including the exon 31, the intron 31 and the exon 32 of ROS1 gene and the exon 19 of EGFR gene, the probes design is performed as follows: two-direction probes dislocation coverage, the probes length was 59 bp, the probes 3'end is modified by Biotin. The probes containing repeat sequences is eliminated by software analysis and experimental results.There are many specific probes sequences. Taking the exon 19 of EGFR gene as an example, Homo sapiens, Release 19 (GRCh37.p13) chr7:552415-55242513 ,four two-direction probes are listed , SEQ ID NO:26-29. Taking the exon 31,the intron 31 and the exon 32 of ROS1 gene as examples, Homo sapiens, Release 19 (GRCh37.p13) chr6:117650492-117658503 , five two-direction probes are listed, SEQ ID NO:35-39. Five software-eliminated two-direction probes containing repeat sequences are listed, SEQ ID NO:40-44. Five two-direction probes with repeated sequences eliminated by the test results are listed, SEQ ID NO:45-49 , shown in the attached table. At the same time, an unbiased probes containing a base free spacer groups is designed for the mutation type NM_005228:exon 19:c.2235_2249del:p.745_750del in the EGFR with exon 19 deleted to increase the capture of the mutant template.The unbiased probes are replaced by the base free spacer groups Spacer 18, which is SEQ ID NO: 50-Spacer 18-SEQ ID NO: 51-3'-Biotin.
wherein, TGAGAAAGTTAAAATTCCCGTCGCTATCAA is SEQ ID NO:50;
CGAAAGCCAACAAGGAAATCCTCGATGTGA is SEQ ID NO:51.

Specifically is,

TGAGAAAGTTAAAATTCCCGTCGCTATCAA-Spacer18-CGAAAGCCAACAAGGAAATC
CTCGATGTGA-3'-Biotin。

Taking 20ng H1650 cell line (NM_005228 :exon19: c.2235_2249del: P.745_750del, the cell line can be purchased from ATCC) , and the library is built with the KAPA kit.

Using hybridization reagents, a hybridization capture of 24h + 24h is performed, using Roche's SeqCap EZ Hybridization and Wash Kit, according to the kit instructions.The sequencing results are as follows.Capture control is performed using a customized commercial capture kit (Roche's SeqCap EZ System customized product).

**Table 4 Effect of the two-direction probes on mutation detection**

| probes coverage | Sample type | Frequency of EGFR c.2235_2249del15 |
|---|---|---|
| two-direction probes | Cell line H1650 | 70.58% |
| Commercial reagents | Cell line H1650 | 38.46% |

It can be seen from the above table that the mutation rate of the two-direction probes dislocation coverage is significantly higher than that of the commercial kit.According to the results of the digital PCR method, the H1650 mutation content is about 62%.This data is said to show that the invention can better hybridize and capture mutated DNA fragments, effectively capture mutation copies, so that mutation detection rate is higher.

### SEQUENCE LISTING

<110> Xiamen Amoy Diagnostics Co.,LTD
<120> probe for nucleic acid enrichment and capture and design method thereof
<130> P14334
<160> 51
<170> Patentln version 3.3
<210> 1
   <211> 59
   <212> DNA
   <213> artificial synthesis
<400> 1
   tggttcaccc agccttccct ggctccctcc ccatttcctc tcatgggcat ttcttctaa 59
<210> 2
   <211> 59
   <212> DNA
   <213> artificial synthesis
<400> 2
   ggctccctcc ccatttcctc tcatgggcat ttcttctaat aaaatctgca gaccatatt 59
<210> 3
   <211> 59
   <212> DNA
   <213> artificial synthesis
<400> 3
   tcatgggcat ttcttctaat aaaatctgca gaccatattg ggtctaatcc catctccag 59
<210> 4
   <211> 59
   <212> DNA
   <213> artificial synthesis
<400> 4
   aaaatctgca gaccatattg ggtctaatcc catctccagt ctgcttcttg gaggaacca 59
<210> 5
   <211> 59
   <212> DNA
   <213> artificial synthesis
<400> 5
   ggtctaatcc catctccagt ctgcttcttg gaggaaccag actaacatga ctctgccct 59
<210> 6
   <211> 59
   <212> DNA
   <213> artificial synthesis
<400> 6
   tggttcaccc agccttccct ggctccctcc ccatttcctc tcatgggcat ttcttctaa 59
<210> 7
   <211> 59
   <212> DNA
   <213> artificial synthesis
<400> 7
   gattagaccc aatatggtct gcagatttta ttagaagaaa tgcccatgag aggaaatgg 59
<210> 8
   <211> 59
   <212> DNA
   <213> artificial synthesis
<400> 8
   aaaatctgca gaccatattg ggtctaatcc catctccagt ctgcttcttg gaggaacca 59
<210> 9
   <211> 59
   <212> DNA
   <213> artificial synthesis
<400> 9
   tgtattatat agggcagagt catgttagtc tggttcctcc aagaagcaga ctggagatg 59
<210> 10
   <211> 59
   <212> DNA
   <213> artificial synthesis
<400> 10
   actaacatga ctctgcccta tataatacaa ataattattt tccatatatc tgattttta 59
<210> 11
   <211> 35
   <212> DNA
   <213> artificial synthesis
<400> 11
   ggccgccctg gtcctggctt tctccggcat catga 35
<210> 12
   <211> 35
   <212> DNA
   <213> artificial synthesis
<400> 12
   ttggtgagtg cacagagccc cagggactcc caagg 35
<210> 13
   <211> 40
   <212> DNA
   <213> artificial synthesis
<400> 13
   gggcaggaag gcaggactga atagtgtctc aggctgtgcc 40
<210> 14
   <211> 48
   <212> DNA
   <213> artificial synthesis
<400> 14
   acaggtgcca aggtgtcact tcgttatgct agtccctgga attgggtg 48
<210> 15
   <211> 35
   <212> DNA
   <213> artificial synthesis
<400> 15
   gggtggtgat tagggcagcc caggccaagc caaaa 35
<210> 16
   <211> 35
   <212> DNA
   <213> artificial synthesis
<400> 16
   ggccgccctg gtcctggctt tctccggcat catga 35
<210> 17
   <211> 36
   <212> DNA
   <213> artificial synthesis
<400> 17
   gctctgtgca ctcaccaatc atgatgccgg agaaag 36
<210> 18
   <211> 35
   <212> DNA
   <213> artificial synthesis
<400> 18
   ttggtgagtg cacagagccc cagggactcc caagg 35
<210> 19
   <211> 35
   <212> DNA
   <213> artificial synthesis
<400> 19
   agtcctgcct tcctgccccc ttgggagtcc ctggg 35
<210> 20
   <211> 40
   <212> DNA
   <213> artificial synthesis
<400> 20
   gggcaggaag gcaggactga atagtgtctc aggctgtgcc 40
<210> 21
   <211> 59
   <212> DNA
   <213> artificial synthesis
<400> 21
   tccttctctc tctgtcatag ggactctgga tcccagaagg tgagaaagtt aaaattccc 59
<210> 22
   <211> 59
   <212> DNA
   <213> artificial synthesis
<400> 22
   ggactctgga tcccagaagg tgagaaagtt aaaattcccg tcgctatcaa ggaattaag 59
<210> 23
   <211> 59
   <212> DNA
   <213> artificial synthesis
<400> 23
   tgagaaagtt aaaattcccg tcgctatcaa ggaattaaga gaagcaacat ctccgaaag 59
<210> 24
   <211> 59
   <212> DNA
   <213> artificial synthesis
<400> 24
   tcgctatcaa ggaattaaga gaagcaacat ctccgaaagc caacaaggaa atcctcgat 59
<210> 25
   <211> 59
   <212> DNA
   <213> artificial synthesis
<400> 25
   gaagcaacat ctccgaaagc caacaaggaa atcctcgatg tgagtttctg ctttgctgt 59
<210> 26
   <211> 59
   <212> DNA
   <213> artificial synthesis
<400> 26
   tccttctctc tctgtcatag ggactctgga tcccagaagg tgagaaagtt aaaattccc 59
<210> 27
   <211> 59
   <212> DNA
   <213> artificial synthesis
<400> 27
   atgttgcttc tcttaattcc ttgatagcga cgggaatttt aactttctca ccttctggg 59
<210> 28
   <211> 59
   <212> DNA
   <213> artificial synthesis
<400> 28
   tcgctatcaa ggaattaaga gaagcaacat ctccgaaagc caacaaggaa atcctcgat 59
<210> 29
   <211> 59
   <212> DNA
   <213> artificial synthesis
<400> 29
   tggaccccca cacagcaaag cagaaactca catcgaggat ttccttgttg gctttcgga 59
<210> 30
   <211> 59
   <212> DNA
   <213> artificial synthesis
<400> 30
   caagtacttt gcaaacacac ataccttatc tcaaggatat agtatgtaat tctacatcc 59
<210> 31
   <211> 59
   <212> DNA
   <213> artificial synthesis
<400> 31
   ataccttatc tcaaggatat agtatgtaat tctacatcca ttatcttcag ctttctccc 59
<210> 32
   <211> 59
   <212> DNA
   <213> artificial synthesis
<400> 32
   agtatgtaat tctacatcca ttatcttcag ctttctccca ctgtattgaa tttttactc 59
<210> 33
   <211> 59
   <212> DNA
   <213> artificial synthesis
<400> 33
   ttatcttcag ctttctccca ctgtattgaa tttttactcc cttctagtaa tttgggaat 59
<210> 34
   <211> 59
   <212> DNA
   <213> artificial synthesis
<400> 34
   ctgtattgaa tttttactcc cttctagtaa tttgggaatg cctggtttat ttgggactc 59
<210> 35
   <211> 59
   <212> DNA
   <213> artificial synthesis
<400> 35
   caagtacttt gcaaacacac ataccttatc tcaaggatat agtatgtaat tctacatcc 59
<210> 36
   <211> 59
   <212> DNA
   <213> artificial synthesis
<400> 36
   ttcaatacag tgggagaaag ctgaagataa tggatgtaga attacatact atatccttg 59
<210> 37
   <211> 59
   <212> DNA
   <213> artificial synthesis
<400> 37
   ttatcttcag ctttctccca ctgtattgaa tttttactcc cttctagtaa tttgggaat 59
<210> 38
   <211> 59
   <212> DNA
   <213> artificial synthesis
<400> 38
   ccctaaagct ggagtcccaa ataaaccagg cattcccaaa ttactagaag ggagtaaaa 59
<210> 39
   <211> 59
   <212> DNA
   <213> artificial synthesis
<400> 39
   cctggtttat ttgggactcc agctttaggg aaaaaaagaa aatattggtt gatatgttt 59
<210> 40
   <211> 59
   <212> DNA
   <213> artificial synthesis
<400> 40
   gggaaaacac ccttcgggat attatccagg agaacttccc caacctagca agacaggcc 59
<210> 41
   <211> 59
   <212> DNA
   <213> artificial synthesis
<400> 41
   gtgttctctg tgtttgccga atttgcatgt tggcctgtct tgctaggttg gggaagttc 59
<210> 42
   <211> 59
   <212> DNA
   <213> artificial synthesis
<400> 42
   acatgcaaat tcggcaaaca cagagaacac cattaagata ctccacgaga agatcaacc 59
<210> 43
   <211> 59
   <212> DNA
   <213> artificial synthesis
<400> 43
   ccttggagaa tctgatgatt atgtgtcttg gggttgatct tctcgtggag tatcttaat 59
<210> 44
   <211> 59
   <212> DNA
   <213> artificial synthesis
<400> 44
   caagacacat aatcatcaga ttctccaagg ttgaaatcaa gtaaaaactg ttaagggca 59
<210> 45
   <211> 59
   <212> DNA
   <213> artificial synthesis
<400> 45
   agaaatgcag catccatttc tatagctcca ggctgtgctt ttcccctgcg ggagccagc 59
<210> 46
   <211> 59
   <212> DNA
   <213> artificial synthesis
<400> 46
   gacaagtctt gggaccaagc tgtccagcct cgctggctcc cgcaggggaa aagcacagc 59
<210> 47
   <211> 59
   <212> DNA
   <213> artificial synthesis
<400> 47
   aggctggaca gcttggtccc aagacttgtc gccacaaccc aacacaccag ctgtggtag 59
<210> 48
   <211> 59
   <212> DNA
   <213> artificial synthesis
<400> 48
   gttagacctg aagaggcact ctggccgctg actaccacag ctggtgtgtt gggttgtgg 59
<210> 49
   <211> 59
   <212> DNA
   <213> artificial synthesis
<400> 49
   cagcggccag agtgcctctt caggtctaac cctcacccat ccttcctcag tgggtgggg 59
<210> 50
   <211> 30
   <212> DNA
   <213> artificial synthesis
<400> 50
   tgagaaagtt aaaattcccg tcgctatcaa 30
<210> 51
   <211> 30
   <212> DNA
   <213> artificial synthesis
<400> 51
   cgaaagccaa caaggaaatc ctcgatgtga 30

### Industrial applicability

The invention can provided probes for nucleic acid enrichment and capture ,can improve the capture specificity , and increase the number of original copies of the nucleic acid captured from the sample,can quickly locate and detect base variation.

## Claims

1. A method for obtaining a set of probes for nucleic acid enrichment and capture, wherein the length of the probes is 30-89 bases, the 3' or 5' end of the probes have biotin labels and can bind to avidin on magnetic beads, comprising the steps of:
(1) selecting the following length of reference sequences containing the gene mutation point in the corresponding position of a reference genome:
if the mutation is a point mutation, insertion mutation or deletion mutation on exons, the exon length is selected;
if the mutation is a fusion mutation, the length of introns and exons at the fusion point is selected;
(2) taking the reference sequences, namely the sense strand template, to design equal length of sense strand probes without overlap between the sense strand probes;
(3) taking the antisense strand template as the reverse complementary sequences of the reference sequences to design antisense strand probes of equal length without overlap, wherein the antisense strand probes without overlap have the same length as the sense strand probes without overlap, and are dislocated with the sense strand probes without overlap, whereby this dislocation comprises that each sense strand and the corresponding antisense strand probes have a complementary part of a half length of probes, wherein the length of the antisense strand probes without overlap is n, wherein
- if n is odd, the dislocation is (n+1) /2; and
- if n is even, the dislocation is n/2;
(4) eliminating the probes containing repeat sequences;
(5) according to high-throughput sequencing results of sample DNA captured by the probes, captured non-specific fragments are compared with all probes, and the probes with matching results with the non-specific fragments are eliminated; whereby
(6) the capture probes which are applicable to the reference sequences are obtained.

2. The method according to claim 1, wherein the probe sequences are: mutation site upstream matching sequences, base free spacer groups and mutation site downstream matching sequences;
wherein the length of the probe sequences is n, and
if n is odd, the length of the upstream matching sequences or the downstream matching sequences is (n+1)/2; and
if n is even, the length of upstream matching sequences or downstream matching sequences is n/2.

3. The method according to claim 2, wherein for the mutation point, the mutation bases at the mutation point are replaced by base free groups in the design of the probes.

4. Use of a set of probes for nucleic acid enrichment and capture selected in the method according to any of claims 1 to 3 with a sequence selected from the group consisting of SEQ ID NOs 6, 7, 8, 9, 10, 26, 27, 28, 29, 45, 46, 47, 48, 49, 50, and 51.

## Patentansprüche

1. Verfahren zum Erhalten eines Satzes von Sonden zur Anreicherung und zum Einfangen von Nukleinsäure, wobei die Länge der Sonden 30-89 Basen beträgt, das 3'- oder 5'-Ende der Sonden Biotinmarkierungen aufweist und an Avidin auf Magnetkugeln bindet, umfassend die folgenden Schritte:
(1) Auswählen der folgenden Länge von Referenzsequenzen, die den Genmutationspunkt an der entsprechenden Position eines Referenzgenoms enthalten:
wenn es sich bei der Mutation um eine Punktmutation, Insertionsmutation oder Deletionsmutation in Exonen handelt, wird die Exonlänge ausgewählt;
wenn es sich bei der Mutation um eine Fusionsmutation handelt, wird die Länge der Introns und Exons am Fusionspunkt ausgewählt;
(2) Benutzen der Referenzsequenzen, nämlich der Sense-Strang-Vorlage, zum Erstellen von Sense-Strang-Sonden gleicher Länge ohne Überlappung zwischen den Sense-Strang-Sonden;
(3) Benutzen der Antisense-Strang-Vorlage als reverse komplementäre Sequenzen der Referenzsequenzen zum Erstellen von Antisense-Strang-Sonden gleicher Länge ohne Überlappung, wobei die Antisense-Strang-Sonden ohne Überlappung die gleiche Länge wie die Sense-Strang-Sonden ohne Überlappung aufweisen und mit den Sense-Strang-Sonden ohne Überlappung verlagert werden, hierbei umfasst diese Verlagerung, dass jeder Sense-Strang und die entsprechenden Antisense-Strang-Sonden einen Komplementaritätsteil der halben Länge der Sonden aufweisen, wobei die Länge der Antisense-Strang-Sonden ohne Überlappung n beträgt, wobei
- wenn n ungerade ist, die Verlagerung (n+1)/2 beträgt; und
- wenn n gerade ist, die Verlagerung n/2 beträgt;
(4) Eliminieren der Sonden, die Wiederholungssequenzen enthalten;
(5) Gemäß Hochdurchsatz-Sequenzierungsergebnissen von durch die Sonden eingefangener Proben-DNA werden eingefangene nicht spezifische Fragmente mit allen Sonden verglichen und die Sonden mit den mit nicht spezifischen Fragmenten übereinstimmenden Ergebnissen werden eliminiert; hierbei
(6) werden die Einfangsonden erhalten, die auf die Referenzsequenzen anwendbar sind.

2. Verfahren nach Anspruch 1, wobei die Sondensequenzen Folgendes sind: Mutationsstelle stromaufwärts von übereinstimmenden Sequenzen, basenfreie Spacergruppen und Mutationsstelle stromabwärts von übereinstimmenden Sequenzen;
wobei die Länge der Sondensequenzen n beträgt und
wenn n ungerade ist, die Länge der übereinstimmenden Sequenzen stromaufwärts oder der übereinstimmenden Sequenzen stromabwärts (n+1)/2 beträgt; und
wenn n gerade ist, die Länge der übereinstimmenden Sequenzen stromaufwärts oder der übereinstimmenden Sequenzen stromabwärts n/2 beträgt.

3. Verfahren nach Anspruch 2, wobei die Mutationsbasen am Mutationspunkt durch basenfreie Gruppen beim Erstellen der Sonden ersetzt werden.

4. Verwendung eines Satzes von Sonden zur Anreicherung und zum Einfang von Nukleinsäure, der in dem Verfahren nach einem der Ansprüche 1 bis 3 mit einer Sequenz ausgewählt wurde, die aus der Gruppe bestehend aus SEQ ID Nr.: 6, 7, 8, 9, 10, 26, 27, 28, 29, 45, 46, 47, 48, 49, 50 und 51 ausgewählt ist.

## Revendications

1. Procédé d'obtention d'un ensemble de sondes pour l'enrichissement et la capture d'acides nucléiques, dans lequel la longueur des sondes est de 30 à 89 bases, l'extrémité 3' ou 5' des sondes comporte des marqueurs de biotine et peut se lier à l'avidine sur des billes magnétiques, comprenant les étapes de :
(1) sélection de la longueur suivante de séquences de référence contenant le point de mutation génique à la position correspondante d'un génome de référence :
si la mutation est une mutation ponctuelle, une mutation par insertion ou une mutation par délétion sur les exons, la longueur de l'exon est sélectionnée ;
si la mutation est une mutation par fusion, la longueur des introns et des exons au point de fusion est sélectionnée ;
(2) utilisation des séquences de référence, à savoir la matrice de brin sens, pour concevoir des sondes de brin sens de longueur égale sans chevauchement entre les sondes de brin sens ;
(3) utilisation de la matrice de brin antisens en tant que séquences complémentaires antisens des séquences de référence pour concevoir des sondes de brin antisens de longueur égale sans chevauchement, les sondes de brin antisens sans chevauchement ayant la même longueur que les sondes de brin sens sans chevauchement, et étant désalignées avec les sondes de brin sens sans chevauchement, ce désalignement impliquant que chaque brin sens et les sondes de brin antisens correspondantes ont une partie complémentaire d'une demi-longueur de sondes, dans laquelle la longueur des sondes de brin antisens sans chevauchement est n, dans lequel
- si n est impair, le désalignement est (n+1)/2 ; et
- si n est pair, le désalignement est n/2 ;
(4) élimination des sondes contenant des séquences répétées ;
(5) en fonction des résultats du séquençage à haut débit de l'échantillon d'ADN capturé par les sondes, les fragments non spécifiques capturés sont comparés à toutes les sondes, et les sondes présentant des résultats correspondants aux fragments non spécifiques sont éliminées ; de sorte que
(6) les sondes de capture qui sont applicables aux séquences de référence sont obtenues.

2. Procédé selon la revendication 1, dans lequel les séquences de sonde sont : des séquences correspondantes en amont du site de mutation, des groupes d'espaceurs sans base et des séquences correspondantes en aval du site de mutation ;
dans lequel la longueur des séquences de sondes est n, et
si n est impair, la longueur des séquences correspondantes en amont ou des séquences correspondantes en aval est (n+1)/2 ; et
si n est pair, la longueur des séquences correspondantes en amont ou des séquences correspondantes en aval est n/2.

3. Procédé selon la revendication 2, dans lequel, pour le point de mutation, les bases de mutation au point de mutation sont remplacées par des groupes sans base dans la conception des sondes.

4. Utilisation d'un ensemble de sondes pour l'enrichissement et la capture d'acides nucléiques sélectionnés dans le procédé selon l'une quelconque des revendications 1 à 3 ayant une séquence choisie dans le groupe constitué par SEQ ID NO : 6, 7, 8, 9, 10, 26, 27, 28, 29, 45, 46, 47, 48, 49, 50 et 51.
